# EUROPÄISCHE PATENTSCHRIFT

(11) **EP 0 293 912 B1**
(45) Veröffentlichungstag und Bekanntmachung des Hinweises auf die Patenterteilung: **25.01.1995**
(21) Anmeldenummer: 88108912.2
(22) Anmeldetag: 03.06.1988
(51) Int. Cl.: C07D 213/04, C07D 215/02

(54) **Verfahren zur Herstellung von gegebenenfalls substituierten Ethansulfobetainverbindungen**
Process for the preparation of ethane sulfobetaine compounds which can be substituted
Procédé pour la préparation de composés d'éthane sulfobétaine le cas échéant substitués

(30) Priorität: 04.06.1987 DE 3718774
(43) Veröffentlichungstag der Anmeldung: 07.12.1988
(73) Patentinhaber: Raschig Aktiengesellschaft, D-67061 Ludwigshafen (DE)
(72) Erfinder: Clauss, Wolfgang, D-6730 Neustadt (DE); Fell, Bernhard, D-5100 Aachen (DE); Hendricks, Günter, D-5100 Aachen (DE); Kurze, Werner, D-6708 Neuhofen (DE); Lämmerzahl, Frank, D-6900 Heidelberg (DE); Wassenberg, Willy, D-6708 Neuhofen (DE)
(74) Vertreter: Hering, Hartmut, Dipl.-Ing.

(56) Entgegenhaltungen:
- DE-A- 1 518 400
- DE-A- 2 310 042
- DE-A- 2 943 266
- GB-A- 2 075 970
- GB-A- 2 081 259
- US-A- 3 200 127
- "ULLMANNS ENCYKLOPAEDIE DER TECHNISCHEN CHEMIE" 4. Auflage, Band 22,Seiten 480-482, 1982.

## Beschreibung

Die Erfindung betrifft ein Verfahren zur Herstellung von gegebenenfalls substituierten Ethansulfo-betainverbindungen.

Betainverbindungen der Ethansulfonsäure entstehen, wenn man das Primärprodukt der Sulfonierung von Olefinen mit Schwefeltrioxid oder dessen Komplexe, z.B.mit 1,4-Dioxan,mit stickstoffhaltigen Verbindungen, z.B. mit Aminen oder Pyridinen umsetzt Als mögliche Zwischenprodukte der Sulfonierung werden 1,2-Sultone (=1,2-Oxathietan - 2,2 - dioxidverbindungen) angenommen, die zu stabileren 1,3-Sultonen und 1,4-Sultonen und insbesondere zu Alkansulfonsäuren isomerisieren. Gleichzeitig können auch noch andere Nebenprodukte, wie z.B. β-Disultone oder homologe Carbylsulfate gebildet werden. (Ullmanns Encyklopädie der technischen Chemie Bd. 22 (1982), Seiten 480 bis 482).

Dieser Weg ist wegen der getrennten Reaktionsstufen der Bildung des SO₃-Komplexes z.B. mit Dioxan und der weiteren Umsetzung mit dem Olefin zum 1,2-Sulton und der weiteren Umsetzung mit dem Betainbildner umständlich und verlustreich.

Verbindungen, die den genannten Ethansulfonsäuren sehr ähnlich sind, lassen sich durch Reaktion von Aminoverbindungen mit 1,3-Propansulton herstellen. 1,3-Propansulton ist aber nicht nur toxisch sondern besitzt gegenüber den 1,2-Sultonen eine erheblich geringere Reaktivität, so dass bei den Umsetzungen mit den Aminoverbindungen höhere Reaktionstemperaturen und längere Reaktionszeiten in Kauf genommen werden müssen. Ebenfalls verhindert die geringere Reaktivität des 1,3-Propansultons in vielen Fällen einen vollständigen Umsatz des 1,3-Sultons, so dass die Endprodukte durch 1,3-Propansultonspuren verunreinigt sein können.

Es wurde gefunden, dass diese Nachteile vermieden werden können, wenn man erfindungsgemäß unter bestimmten, an sich bekannten Bedingungen die Olefinsulfonierung in Gegenwart des Betainbildners durchführt, wobei eine ganze Palette wertvoller oberflächenaktiver Substanzen erhalten werden kann.

Das erfindungsgemäße Verfahren zur Herstellung von gegebenenfalls substituierten Ethansulfo-betainverbindungen der allgemeinen Formel 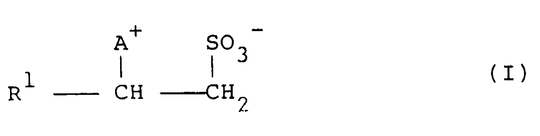
in der R¹ eine C₁ - C₂₀ - Alkylgruppe und A ein tertiäres aliphatisches Amin mit 2 bis 20 C-Atomen oder ein gegebenenfalls mit 1 bis 3 C₁- bis C₄ - Alkylgruppen oder C₂ - bis C₄ - Alkenylgruppen oder 1 oddr 2 Phenylgruppen substituierter heterocyclischer Rest mit 5 oder 6 Atomen, von denen mindestens enes ein Stickstoffatom ist und die anderen Kohlenstoff-, Stickstoff-, Schwefel- und/oder Sauerstoffatome sind wobei dieser Rest mit ein oder zwei aromatischen oder heterocyclischen Resten mit 5 oder 6 Atomen kondensiert sein kann, darstellt, ist dadurch gekennzeichnet, dass man ein Olefin der allgemeinen Formel

R¹ ― CH = CH₂ (II)

in der R¹ die genannte Bedeutung hat, sowie eine Verbindung der allgemeinen Formel A, in der A die genannte Bedeutung hat bei einer Temperatur von +20°C bis -78°C mit Schwefeltrioxid in einem inerten Lösungsmittel oder Lösungsmittelgemisch umsetzt.

Bevorzugte Alkylgruppen R¹ sind einmal C₁ bis C₅-Niederalkylgruppen und andererseits C₁₄ bis C₁₈-Alkylgruppen, wobei die Alkylgruppen gradkettig oder verzweigt sein können. Vorzugsweise handelt es sich um Verzweigungen, die Methyl-, Ethyl- oder Propylgruppen darstellen, welche an eine gradkettige höhere Alkylgruppe gebunden sind. Die Verzweigungen dürfen jedoch nicht an der Doppelbindung oder in α-Stellung der Doppelbindung stehen. Bevorzugte Olefine sind Propen, Buten-1, cis- und trans -n-Buten und die n-α-C₅-C₂₀- Olefine.

Als Reste A sind Pyridin-, Picolin, Piperidin-, Morpholin- und Lutidinreste bevorzugt. Es sind aber auch Imidazolin-, Pyrol-, Imidazol-, Chinolin- und Isochinolinreste, sowie Reste des Thiazols geeignet.

Die erfindungsgemäß in hoher Ausbeute erhaltenen 1,2-Ethansulfo-betainverbindungen können in hoher Reinheit und Ausbeute erhalten werden, ohne dass das 1,2-Ethan-sulton als Zwischenprodukt isoliert wird oder ohne Isolieren weiter umgesetzt wird. ("Eintopfverfahren").

Die erfindungsgemäße Umsetzung erfolgt unter Einhaltung möglichst milder Reaktionsbedingungen, d.h. bei den genannten tiefen Temperaturen und unter Verwendung inerter Lösungsmittel. Letzteres ist insbesondere notwendig bei Einsatz von niedermolekularen, unter Normalbedingungen gasförmigen Olefinen, wie z.B. Propen und Buten-1. Als inerte Lösungsmittel können z.B. Alkane oder auch chlorierte Kohlenwasserstoffe, wie Dichlormethan oder Dichlorethan verwendet werden. SO₃ wird gasförmig mit Luft, Stickstoff oder anderen inerten Gasen verdünnt oder ebenfalls vorzugsweise in geeigneten Lösungsmitteln, wie Dichlormethan und Dichlorethan gelöst eingesetzt. Außer mit freiem SO₃ und zur Erreichung eines möglichst hohen 1,2-Sultonanteils im Primärprodukt der Olefinsulfonierung auch mit durch Dioxan oder anderen Verbindungen komplexierten und damit in seiner Reaktivität abgestuften SO₃ gearbeteite werden.

Das erfindungsgemäße Verfahren wird halbkontinuierlich im batch-Reaktor, vorteilhafterweise kontinuierlich in Dünnschichtreaktoren bekannter Bauart (Fallfilmreaktor, TO-Reaktor, a.a.O.) ausgeführt. Das Molverhältnis Olefin : SO₃ beträgt 1 : 2 bis 2 : 1, vorzugsweise 1 : 1.

Ein besonderer Vorteil der nach dem erfindungsgemäßen Verfahren hergestelltenproduktesind die - verglichen mit anderen Herstellungsverfahren - niedrigen Herstellungskosten.

Die Produkte fallen z.B. mineralsalzfrei und elektrolytfrei an, wodurch ihre Reindarstellung und Anwendung bedeutend erleichert wird.

Die große Variationsbreite der Verbindungen gemäß der Erfindung durch gezielte Wahl der Reste R¹, R² und A erlaubt eine optimale Anpassung der grenzflächenaktiven and anwendungstechnischen Eigenschaften der Produkt für spezielle Leistungsanforderungen z. B. bei Wasch- und Reinigungsmitteln, Kosmetika, Toilettenerzeugnissen und sonstigen sog. personal care products, Textilhilfsmitteln, Emulgatoren für die verschiedensten Zwecke, Reinigungsmittel für die Metallindustrie, bei Tensiden für die Erdölaufarbeitung (Desmulgatoren), bei Tensiden für die Chemie- und Papierindustrie oder als Sammler und Schäumer in der Flotationstechnik (Surfactants in Consumer Products, Springer-Verlag, Berlin-Heidelberg, New York 1987).

Insbesondere können die Sulfobetaine, die sich von Pyridin oder Pyridinabkömmlingen herleiten, durch gezielte Variation der Reste R¹ und A gewünschten anwendungstechnischen Eigenschaften optimal angepaßt werden. Sie werdenals Tenside mit guter Hartwasserbeständigkeit, Unempfindlichkeit gegenüber Elektrolyten, (wichtig u.a. für den einsatz bei der tertiären Erdölföderung) guter Haut- und Schleimhautverträglichkeit, Kompatibilität mit anderen ionischen und nichtionischen Tensiden sowie antistatischen und textilweichmachenden Eigenschaften eingesetzt.

Diese Tensidklasse ist in Haushaltsprodukten ebenso wie in Erzeugnissen für Chemie und Technik - verglichen mit vielen anderen Tensiden mit verhältnismäßig hohen Gestehungskosten - wegen der geringeren Herstellungskosten durch das erfindungsgemäße Verfahren von besonderem Interesse.

Die Erfindung wird durch folgende Beispiele näher erläutert. Hierin ist RT = Raumtemperatur = ca. 20°C

### Beispiel 1

### 1-(1-n-Propyl-2-sulfoethyl)pyridinium-betain

Eine Lösung von 24.3 g (0,35 mol) n-Penten-1 und 27.7 g (0,35 mol) Pyridin in 200 ml 1.2-Dichlorethan wird auf O°C gekühlt und danach eine Lösung aus 27.3 (0,35 mol) SO₃ in 100 ml-Dichlorethan so zugetropft, dass die Reaktionstemperatur + 10°C nicht überschreitet. Nach der SO₃-Zugabe wird noch eine Stunde bei 0°C gerührt, das ausgefallene 1-(1-n-Propyl-2-sulfoethyl)pyridinium-betain abfiltriert und aus Ethanol/Essigester (1/1) umkristallisiert. Nach der Filtration und Trocknung erhält man das Produkt in Form weißer Kristalle.

Elementaranalyse und ¹H-NMR-Spektroskopie zeigen die Bildung der folgenden Verbindung an:

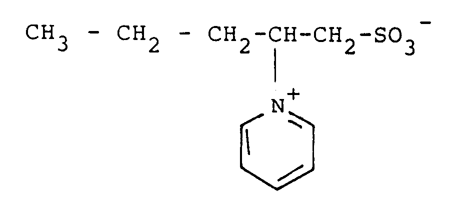

| Elementaranalyse: | | | | |
|---|---|---|---|---|
| berechnet: | 52,39%C | 6,59% H | 6,15% N | 13,98% S |
| gefunden: | 52,5 %C | 6,7 % H | 6,2 % N | 14,0 % S |

### Beispiel 2

### 1-(1-n-Propyl-2-sulfoethyl)-chinolium-betain

Eine Lösung von 27 g (0,38 mol) n-Penten-(1) und 49,7 g (0,38 mol) Chinolin in 200 ml 1,2-Dichlorethan wird auf O°C gekühlt und danach eine Lösung aus 30,9 g (0,38 mol) SO₃ in 100 ml 1,2-Dichlorethan so zugesetzt, dass die Reaktionstemperatur -10°C nicht überschreitet. Nach der SO₃ - Zugabe wird noch ein paar Stunden gerührt. Im Gegensatz zu Beispiel 1 fällt kein Produkt aus, sondern nach Abziehen des Lösungsmittels bleibt eine hochviskose Flüssigkeit zurück, in in polaren Lösungsmitteln, wie MeOH oder Wasser gut löslich ist.

### Beispiel 3

### 1-(1-n-Propyl-2-sulfoethyl)-picolinium-betain

Gemäß Beispiel 1 werden folgende Verbindungen umgesetzt:
20 g (0,28 mol) n-Penten-(1)
26,5 g (0,28 mol) Picolin in
150 ml 1,2-Dichlorethan als Lösungmittel
22,9 g (o,28 mol) SO₃ in
100 ml 1,2-Dichlorethan als Lösungsmittel
Es wurde gemäß IR und¹H-NMR-Spektroskopie folgende Verbindung erhalten:
Ausbeute: 30 g = 43,5 %

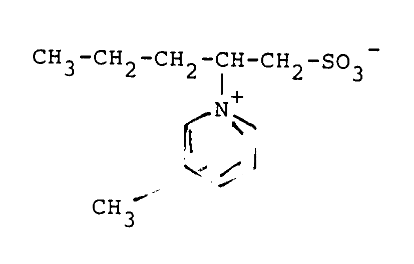

## Patentansprüche

1. Verfahren zur Herstellung von gegebenenfalls substituierten Ethansulfo-betainverbindungen der allgemeinen Formel 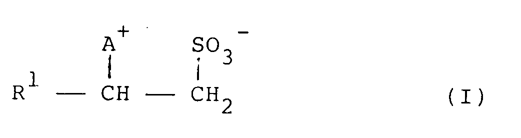 in der R¹ eine C₁ - C₂₀ - Alkylgruppe bedeutet, und A ein tertiäres aliphatisches Amin mit 2 bis 20 C-Atomen oder ein gegebenenfalls mit 1 bis 3 C₁- bis C₄-Alkylgruppen oder C₂- bis C₄-Alkenylgruppen oder 1 bis 2 Phenylresten substituierter heterocyclischer Rest mit 5 bis 6 Atomen, von denen mindestens eines ein Stickstoffatom ist und die anderen Kohlenstoffstickstoff-, Schwefel- und/oder Sauerstoffatome sind, wobei dieser Rest mit ein oder zwei aromatischen oder heterocyclischen Resten mit 5 oder 6 Atomen kondensiert sein kann, darstellt, **dadurch gekennzeichnet**, daß man ein Olefin der allgemeinen Formel
R¹ ― CH = CH₂ (II)
in der R¹ die genannte Bedeutung hat, sowie eine Verbindung der allgemeinen Formel A mit der genannten Bedeutung bei einer Temperatur von +20°C bis -78°C mit Schwefeltrioxid in einem inerten Lösungsmittel oder Lösungsmittelgemisch in einer Eintopfreaktion umsetzt.

2. Verfahren nach Anspruch 1, **dadurch gekennzeichnet**, dass man die Umsetzung bei einer Temperatur von +10°C bis -20°C durchführt.

3. Verfahren nach Anspruch 1 und 2, **dadurch gekennzeichnet**, dass man die Umsetzung in einem C₅ - C₁₅- Alkan oder einem C₁- bis C₅ - chlorierten Alkan als Lösungsmittel durchführt.

4. Verfahren nach den Ansprüchen 1 - 3, **dadurch gekennezeichnet**, dass man die Umsetzung in 1,4-Dioxan als Co-Lösungsmittel durchführt.

5. Verwendung nach dem Verfahren gemäß den Ansprüchen 1 oder 4 hergestellten Verbindungen als oberflächenaktive Mittel.

## Claims

1. Process for preparing optionally substituted ethane sulphobetaine compounds of the general formula 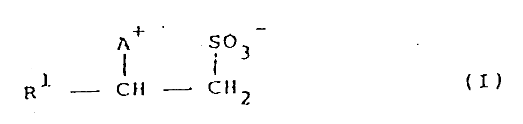 in which R¹ denotes a C₁ - C₂₀ alkyl group, and A is a tertiary aliphatic amine having 2 to 20 C atoms or a heterocyclic radical having 5 to 6 atoms, of which at least one is a nitrogen atom and the others are carbon, nitrogen, sulphur and/or oxygen atoms, optionally substituted by 1 to 3 C₁ to C₄ alkyl groups or C₂ to C₄ alkenyl groups or 1 to 2 phenyl radicals, it being possible for this radical to be condensed with one or two aromatic or heterocyclic radicals having 5 or 6 atoms, characterised in that an olefin of the general formula
R¹ ― CH = CH₂ (II)
in which R¹ has the said meaning, and a compound of the general formula A having the said meaning is reacted with sulphur trioxide at a temperature of +20°C to -78°C in an inert solvent or solvent mixture in a one-pot reaction.

2. Process according to claim 1, characterised in that the reaction is carried out at a temperature of +10°C to -20°C.

3. Process according to claim 1 and 2, characterised in that the reaction is carried out in a C₅ - C₁₅ alkane or a C₁ to C₅ chlorinated alkane as solvent.

4. Process according to claims 1 - 3, characterised in that the reaction is carried out in 1,4-dioxane as co-solvent.

5. Use of compounds prepared by the process according to claims 1 or 4 as surface-active agents.

## Revendications

1. Procédé de préparation de composés éthanesulfobétaïne éventuellement substitués, de formule générale 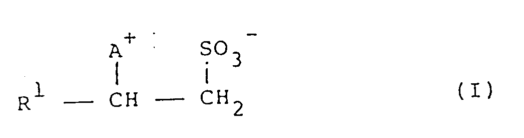 dans laquelle R¹ représente un groupe alkyle en C₁-C₂₀ et A représente une amine aliphatique tertiaire ayant de 2 à 20 atomes de C, ou un reste hétérocyclique éventuellement substitué par 1 à 3 groupes alkyle en C₁-C₄ ou alcényle en C₂-C₄ ou 1 à 2 restes phényle, le reste hétérocyclique comportant 5 à 6 atomes, dont au moins un est un atome d'azote et les autres sont des atomes de carbone, azote, soufre et/ou oxygène, ce reste pouvant être condensé avec un ou deux reste(s) aromatique(s) ou hétérocyclique(s) comportant 5 ou 6 atomes, caractérisé en ce que l'on fait réagir une oléfine de formule générale
R¹ ― CH = CH₂ (II)
dans laquelle R¹ a la signification mentionnée ci-dessus, ainsi qu'un composé de formule A ayant la signification mentionnée ci-dessus, à une température de +20°C à -78°C, avec du trioxyde de soufre, dans un solvant ou mélange de solvants inerte, la réaction se déroulant dans un récipient unique.

2. Procédé selon la revendication 1, caractérisé en ce que l'on effectue la réaction à une température de +10°C à -20°C.

3. Procédé selon les revendications 1 et 2, caractérisé en ce que l'on effectue la réaction dans un alcane en C₅-C₁₅ ou dans un alcane en C₁-C₅ chloré, utilisé en tant que solvant.

4. Procédé selon les revendications 1 à 3, caractérisé en ce que l'on effectue la réaction dans du 1,4-dioxane utilisé en tant que co-solvant.

5. Utilisation des composés préparés selon le procédé revendiqué dans la revendication 1 ou 4, en tant qu'agent tensioactif.
